# EUROPÄISCHE PATENTSCHRIFT

(11) **EP 1 256 571 B1**
(45) Veröffentlichungstag und Bekanntmachung des Hinweises auf die Patenterteilung: **02.08.2006**
(21) Anmeldenummer: 02011911.1
(22) Anmeldetag: 25.10.1996
(51) Int. Cl.: C07C 323/58, C07C 319/28

(54) **Verfahren zur Herstellung von D,L-Methionin oder dessen Salz**
Process for the preparation of D,L-methionine or a salt thereof
Procédé de préparation de la D,L-méthionine ou de son sel

(30) Priorität: 18.12.1995 DE 19547236
(43) Veröffentlichungstag der Anmeldung: 13.11.2002
(62) Teilanmeldung aus: 96117124.6
(73) Patentinhaber: Degussa AG, 40474 Düsseldorf (DE)
(72) Erfinder: Geiger, Friedhelm, Dr., 63526 Erlensee (DE); Halsberghe, Baudouin, Mobile, Alabama (US); Hasselbach, Hans Joachim, Dr., 63571 Gelnhausen (DE); Hentschel, Klaus, Dr., 63517 Rodenbach (DE); Huthmacher, Klaus, Dr., 63584 Gelnhausen (DE); Körfer, Martin, 2920 Kalmthout-Achterbroek (DE); Mannsfeld, Sven, Dr., 91207 Lauf/Pegnitz (DE); Tanner, Herbert, Dr., 63457 Hanau (DE); Theissen, Ferdinant, Dr., 53332 Bornheim (DE); Vanrobaeys, Jose, Mobile, Alabama (US); Willigerodt, Klaus, Dr., 53225 Bonn (DE)

(56) Entgegenhaltungen:
- EP-A- 0 143 218
- DE-A- 1 620 332
- DE-A- 1 906 405
- DE-A- 2 106 763
- GB-A- 1 186 538
- CHEMICAL ABSTRACTS, vol. 120, no. 13, 28. März 1994 (1994-03-28) Columbus, Ohio, US; abstract no. 164902t, TERASAWA T. ET AL.: "Preparation of methionine" Seite 1264; Spalte 2; XP002029147 & JP 05 286926 A (SUMITOMO CHEMICAL CO.) 2. November 1993 (1993-11-02)
- DATABASE WPI Section Ch, Week 197409 Derwent Publications Ltd., London, GB; Class B03, AN 1974-16845V XP002205882 & NL 7 311 018 A (ASAHI KASEI KOGYO KK), 12. Februar 1974 (1974-02-12)
- CHEMICAL ABSTRACTS, vol. 84, no. 7, 16. Februar 1976 (1976-02-16) Columbus, Ohio, US; abstract no. 44666k, TAKAGI K. ET AL.: ".alpha.-Amino acids" Seite 556; Spalte 2; XP002033766 & JP 50 106901 A (SUMITOMO CHEMICAL CO., LTD.) 22. August 1975 (1975-08-22)
- CHEMICAL ABSTRACTS, vol. 85, no. 7, 16. August 1976 (1976-08-16) Columbus, Ohio, US; abstract no. 47049t, KOJIMA T. ET AL.: "DL-Methionine alkali salts" Seite 577; Spalte 1; XP002033767 & JP 50 157318 A (KANEBO LTD.) 19. Dezember 1975 (1975-12-19)
- DATABASE WPI Section Ch, Week 9122 Derwent Publications Ltd., London, GB; Class A41, AN 91-159964 XP002033769 MIZUNO T. ET AL.: "Production of alpha-amino acid" & JP 03 095145 A (SUMITOMO CHEM IND KK), 19. April 1991 (1991-04-19)
- DATABASE WPI Section Ch, Week 9122 Derwent Publications Ltd., London, GB; Class A41, AN 91-159965 XP002033770 MIZUNO T. ET AL.: "Production of alpha-amino acid" & JP 03 095146 A (SUMITOMO CHEM IND KK), 19. April 1991 (1991-04-19)
- CHEMICAL ABSTRACTS, vol. 117, no. 17, 26. Oktober 1992 (1992-10-26) Columbus, Ohio, US; abstract no. 172112p, MIZUNO T. ET AL.: "Preparation of methionine" Seite 914; Spalte 1; XP002033768 & JP 04 169570 A (SUMITOMO CHEMICAL CO., LTD.) 17. Juni 1992 (1992-06-17)
- DATABASE WPI Section Ch, Derwent Publications Ltd., London, GB; Class A25, AN 73-64462U XP002033772 & JP 48 014619 A (KANEGAFUCHI CHEMICAL INDUSTRIES CO., LTD.), 23. Februar 1973 (1973-02-23)

## Beschreibung

Die Erfindung betrifft ein Verfahren zur Herstellung von D,L-Methionin oder einem Salz des D,L-Methionins, ausgehend von den Komponenten 3-Methylmercaptopropionaldehyd, Cyanwasserstoff, Ammoniak und Kohlendioxid bzw. 5-(2-Methylmercaptoethyl)-hydantoin bzw. einem Salz des Methionins oder von solchen Komponenten, aus denen die vorgenannten Komponenten herstellbar sind, ggf. in Gegenwart von Wasser.

Die Syntheseschritte lassen sich durch folgende Reaktionsgleichungen veranschaulichen:
5-(2-Methylmercapto)-hydantoinbildung: 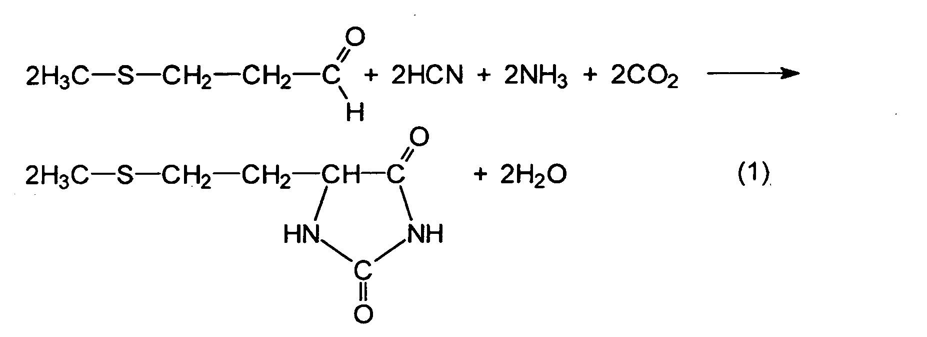
Salzbildung des D,L-Methionins: 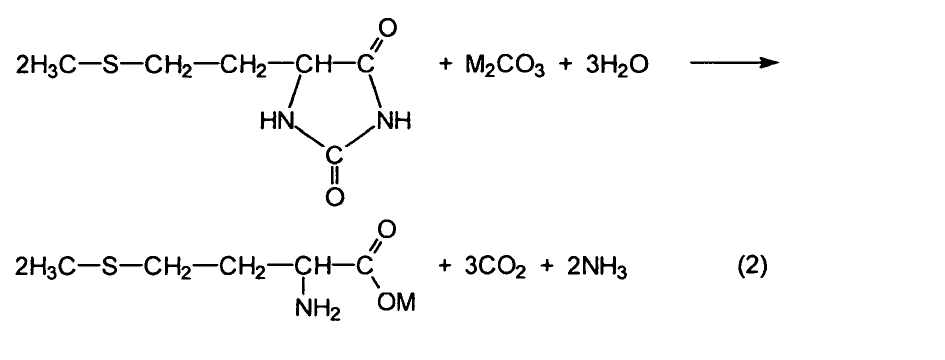
D,L-Methionin-Freisetzung:

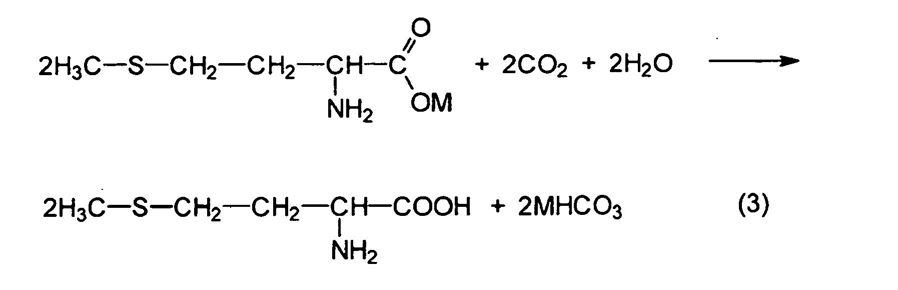

M steht für Alkali, Erdalkali, Ammonium, insbesondere Kalium.

Die Verfahrensstufen 5-(2-Methylmercaptoethyl)-hydantoin-Bildung, Methioninat-Bildung sowie Methionin-Freisetzung können für sich vorteilhaft kontinuierlich betrieben werden und werden zweckmäßigerweise hintereinander verschaltet in einen insbesondere gesamtkontinuierlich verlaufenden Prozeß integriert.

Besonders vorteilhaft werden die Komponenten Ammoniak und Kohlendioxid entsprechend den Prozeßmöglichkeiten recycliert, d. h. in die zuvor ablaufenden Verfahrensstufen wieder eingesetzt. Insbesondere bei der Verwendung von Kalium werden möglichst die gesamten Alkali enthaltenden Agentien in den Prozeß zurückgeführt.

Die 5-(2-Methylmercapto)-Hydantoin-Bildung ist grundsätzlich bekannt. Generell wird dabei entweder von den oben unter 1) beschriebenen Komponenten ausgegangen oder von solchen Komponenten, aus denen diese Komponenten herstellbar sind. Diese sind insbesondere Alkali oder Ammoniumsalze bei den Komponenten Cyanwasserstoff, Ammoniak und Kohlendioxid bzw. Acrolein und Methylmercaptan bei der Komponente 3-Methylmercaptopropionaldehyd, wie sie weiter unten beschrieben werden. So beschreibt die Chem. Rev. 46 (1959) 422 - 425 die Herstellung substituierter Hydantoine durch Umsetzung der entsprechenden Aldehyde und Ketone mit Alkalicyaniden und Ammoniumcarbonat. Die Umsetzung wird entweder unter Anwendung stöchiometrischer Anteile der Substanzen bei 80 °C und 3 bar oder unter Anwendung des Mehrfachen des stöchiometrischen Anteils an Ammoniak bei Temperaturen bis 60 °C und Normaldruck (DT-PS 11 66 201) ausgeführt. Es ist auch bekannt, aus 3-Methylmercaptopropionaldehyd, Ammoniumcarbonat und Cyaniden das 5-(2-Methylmercaptoethyl)-hydantoin herzustellen. Die Umsetzung wird anfangs bei 40 - 120 °C ausgeführt, das Umsetzungsgemisch wird dann auf einen pH-Wert unter 4 eingestellt und die Reaktion bei 50 - 100 °C beendet (US-PS 2 557 913). Weiterhin ist bekannt, das 5-(2-Methylmercaptoethyl)-hydantoin herzustellen, indem man eine Lösung vorlegt, die durch Auflösung von 3-Methylmercaptopropionaldehyd in einer wäßrigen Lösung vom Ammoniak, Kohlendioxid und Cyanwasserstoffsäure oder deren Salze hergestellt wurde und in der ggf., teilweise oder ganz, eine Umsetzung zum Hydantoin erfolgt ist, und in diese Lösung eine wäßrige Lösung von Ammoniak, Kohlendioxid und Cyanwasserstoffsäure oder deren Salze und davon getrennt den 3-Methylmercaptopropionaldehyd einträgt und die Umsetzung durch Erwärmen der Mischung auf bis zu 100 °C bei Normaldruck ausführt (DT-OS 16 20 332). In der japanischen Patentschrift JP 48-005763 wird 3-Methylmercaptopropionaldehyd mit Cyanwasserstoff oder dessen Salze und Ammoniumcarbonat in Gegenwart von Ammoniak und bei 80 °C in 1,5 Stunden zu 5-(2-Methylmercaptoethyl)-hydantoin in 98,5 %iger Ausbeute umgesetzt. Zugesetzte Metallionenkomplexierungsmittel führen in Gegenwart von Wasser zu 97,8 % Ausbeute (JP 48-004465). Eine analoge Umsetzung wird in Gegenwart von organischen Lösungsmitteln bei 50 - 200 °C unter Druck in der Flüssigphase in der Patentschrift JP 40-36676 beschrieben. Eine Eintopfvariante ausgehend von Acrolein, Methylmercaptan, Cyanwasserstoff und Ammoniumcarbonat in Wasser führt bei 50 - 70 °C innerhalb von 2 Stunden zu einem Hydantoin, das zu D,L-Methionin verseift wird (JP 50-004018).

Ähnlich verläuft die in der JP 52-027768 beschriebene Reaktion, jedoch unter Zusatz von Aminosäuren wie Methionin, Threonin, Glycin, Alanin oder Leucin. 3-Methylmercaptopropionaldehyd, Kohlendioxid, Ammoniak, Cyanwasserstoff und kaustisches Alkali führen bei 80 °C innerhalb von 2 Stunden zu 97 % 5-(2-Methylmercaptoethyl)-hydantoin (JP 50-018467).

3-Methylmercaptopropionaldehyd, Natriumcyanid und Ammoniumcarbonat in Wasser ergeben in Gegenwart von Kaliumthiosulfat oder Kaliumcarbonat 5-(2-Methylmercaptoethyl)-hydantoin (SU 740770). Bei der Einstufenumsetzung von Acrolein mit Methylmercaptan, Cyanwasserstoff und Ammoniumcarbonat entsteht in 85 %iger Ausbeute 5-(2-Methylmercaptoethyl)-hydantoin (Asahi Chem. Ind., Agric. Biol. Chem. 52, 589 (1988). Auch eine Einstufenreaktion beschreibt die chinesische Patentschrift CN 85 1085905, jedoch unter Zusatz von Methionin in Essigsäure mit 91 % Umsetzung zum 5-(2-Methylmercaptoethyl)-hydantoin.

Ein nach den bekannten Verfahren hergestelltes 5-(2-Methylmercaptoethyl)-hydantoin enthält in erheblichem Umfang Verunreinigungen durch 5-(2-Methylmercaptoethyl)-hydantoinsäure, 5-(2-Methylmercaptoethyl)-hydantoinsäureamid, Methioninamid, Methioninnitril sowie Methylmercaptopropionaldehydcyanhydrin, Iminonitril und Polymere. Während die drei zuerst genannten Verbindungen bei der alkalischen Hydrolyse wie das Hydantoin in Methionin überführt werden, gelangen die übrigen Verbindungen bzw. deren Verseifungsprodukte in die Verseifungslösung und in das später zu isolierende Methionin, wo sie sich nur sehr schwierig abtrennen lassen. Dies ist um so mehr der Fall, wenn das Methionin aus dem Hydantoin hergestellt und mittels Kohlendioxid aus dem Umsetzungsgemisch abgeschieden und die Mutterlauge im Kreislauf geführt wird. Das erhaltene Methionin ist verfärbt und zeigt eine schlechte Lagerbeständigkeit.

Die alkalische Hydrolyse von 5-(2-Methylmercaptoethyl)-hydantoin ist seit langem bekannt. So beschreiben die US-A 2,527,366 und US-A 2,557,913 die Hydrolyse von 5-(2-Methylmercaptoethyl)-hydantoin in einer wäßrigen Bariumhydroxidlösung unter Druck und erhöhter Temperatur. Diese Verfahren erfordern aber erhebliche Mengen an teurem Bariumhydroxid, außerdem muß das Barium als Neutralsalz wieder abgetrennt werden.

Aus der US-A 2,557,920 ist bekannt, daß α-Aminosäuren durch Verseifung von Hydantoinen unter Verwendung von Natriumhydroxid entstehen. Bei diesen Verfahren werden aber mindestens 3 Mol Natriumhydroxid je Mol Hydantoin benötigt. Analog verhält es sich beim Einsatz von Kaliumhydroxid.

Ferner ist aus der US-A 4,272,631 bekannt, daß ein Gemisch aus Alkali- und Erdalkalihydroxid zur Verseifung von 5-(2-Methylmercaptoethyl)-hydantoin verwendet werden kann. Allerdings müssen bei diesen Verfahren die Erdalkaliionen bei der Freisetzung von Methionin erst abgetrennt werden, so daß nur Ausbeuten von max. 80,5 % erreicht werden.

In der US-A 4,259,925 wird die Hydrolyse von 5-(2-Methylmercaptoethyl)-hydantoin unter Druck bei 105 bis 230 °C in einem Medium, das ein Metallhydroxid und einen Alkohol mit einem Siedepunkt von 125 bis 130 °C enthält, durchgeführt. Nachteilig ist, daß der hochsiedende Alkohol wieder zurückgewonnen werden muß. Außerdem beträgt die Ausbeute nur 65 %.

In der DE-PS 19 06 405 wird die Hydrolyse von 5-(2-Methylmercaptoethyl)-hydantoin unter Verwendung einer wässrigen Lösung von Alkalicarbonat und/oder Alkalihydrogencarbonat beschrieben. Während der Hydrolyse werden Ammoniak und Kohlendioxid laufend entfernt. Von den Alkalicarbonaten wird Kaliumcarbonat bevorzugt; es wird ein Mol-Verhältnis Hydantoin zu Alkali von 1 : 1 bis 1 : 5 angewendet. Die Hydrolyse wird unter Druck bei 120 bis 220 °C durchgeführt. Die kontinuierliche Druckapparatur besteht aus drei aufwendig hintereinander geschalteten Umlaufverdampfern. Die Alkalimethioninatlösung wird zur Freisetzung von D,L-Methionin mit Kohlendioxid verwendet; die Mutterlauge aus der Abtrennung des auskristallisierten Methionins wird im Kreislauf ggf. mit Ausschleusung von 1 - 2 % wieder zur Hydrolyse des Hydantoins eingesetzt.

Die DE-AS 15 18 339 beschreibt ein Verfahren, bei dem bei der Hydrolyse entstandene gasförmige Reaktionsprodukte (Ammoniak und Kohlendioxid) aus der Reaktion entfernt werden, um das Reaktionsgleichgewicht in Richtung der Aminosäure zu verschieben, wodurch die Ausbeute gesteigert wird. Um dies zu erreichen, ist jedoch eine komplexe apparative Anordnung zur Druckregelung der Gasströme erforderlich.

Die japanische Patentschrift 49/116 008 beschreibt ein Verfahren, bei dem die Hydrolyse von 5-(2-Methylmercaptoethyl)-hydantoin in Gegenwart von Vanadinsäuren, Molybdänsäuren, Wolframsäuren oder deren Derivate durchgeführt wird. Die Ausbeuten betragen ca. 70 %. Schwierig ist die Abtrennung des Katalysators. Zur Herstellung einer hochkonzentrierten Methionin-haltigen Lösung muß Alkali, z. B. eine Kaliumverbindung, zugesetzt werden.

Aus der japanischen Patentanmeldung 75/106 901 (C. A. 84, 44666k (1976)) ist bekannt, daß Methionin durch Hydrolyse von 5-(2-Methylmercaptoethyl)-hydantoin in Gegenwart von etwa 1,2 eq Natriumhydroxid und von etwa 9 eq Ammoniak bei 180 °C hergestellt wird. Die bei dieser Arbeitsweise intermediär entstehende Natriummethioninat-Lösung enthält jedoch zwangsläufig neben dem Natriummethioninat auch Natriumcarbonat, das bei dieser Reaktionsführung ausfällt und deshalb insbesondere bei einem kontinuierlichen Prozeß störend ist. Analoges gilt bei der Verwendung von Kaliumhydroxid sowie für die Verfahrensweise gemäß DE-PS 19 06 405.

Die DE 26 14 411 A beschreibt die Hydrolyse von 5-(2-Methylmercaptoethyl)-hydantoin mit Wasser in Gegenwart von Imidazol bei 160 °C. Die Ausbeuten sind niedrig und auch hier muß eine Alkaliverbindung zur Erzeugung einer hohen Lösungskonzentration zugegeben werden.

Die japanischen Patentanmeldungen JP 03/95145 und JP 03/95146 beschreiben die Hydrolyse von Hydantoinen mit Wasser bei erhöhter Temperatur und erhöhtem Druck in Gegenwart von Metalloxiden oder -oxidgemischen, wie z. B. ZrO₂, TiO₂, Nb₂O₅ oder TiO₂-Nb₂O₅; die Ausbeuten liegen allerdings nur bei 65 bis 66 %. Diese Lösungen müssen mit einer Alkaliverbindung neutralisiert werden.

All diese Verfahren bringen entweder geringe Ausbeuten oder haben den Nachteil, daß Methionin oder Salze wie Carbonate während des Prozesses ausfallen, wodurch weitere Verfahrensschritte verursacht werden und insbesondere großtechnische Verfahren, ganz besonders kontinuierliche Verfahren, kaum möglich sind.

Die Freisetzung des Methionins aus dem Alkalisalz ist allgemein bekannt. Nach dem Prinzip, starke Säuren setzen schwächere Säuren aus ihren Salzen frei, wird z. B. mit Salzsäure, Schwefelsäure, Phosphorsäure oder einem stark sauren Ionenaustauscher freies D,L-Methionin ausgefällt (DE 21 40 506 C; DE 21 22 491 C; DE 29 12 066 A; BE 877 200, US-A 3 433 832, FR 1 532 723). Allerdings muß dann noch das als Nebenprodukt anfallende Alkalisalz abgetrennt werden. Da die eingesetzte Säure im allgemeinen nicht mehr zurückgewonnen wird, ist diese Vorgehensweise für ein kontinuierliches, wirtschaftliches und umweltschonendes Herstellverfahren ungeeignet.

Zweckmäßigerweise wurde deshalb - wie beispielsweise in den Patentschriften US-A 2 557 913, DE-PS 19 06 405 und JP 42/44056 beschrieben - D,L-Methionin aus den Hydrolyselösungen des 5-(2-Methylmercaptoethyl)-hydantoins mit Kohlendioxid in wässriger Lösung ausgefällt. Bei dieser Methode fällt i. d. R. das D,L-Methionin in Form von dünnen Plättchen oder schuppenförmig an. Dies bedingt eine schlechte Filtrierbarkeit des Produkts und behindert ein Auswaschen des Kristallkuchens; außerdem fällt ein D,L-Methionin mit schlechtem Fließverhalten und Neigung zur Verklumpung an. Um diesen Nachteilen entgegenzuwirken, werden gemäß der japanischen Patentschrift JP 42/44056 Zusätze wie Kasein oder wasserlösliche, hochmolekulare Cellulosederivate bei der Methioninfällung mit Kohlendioxid zugesetzt.

Aufgabe der vorliegenden Erfindung ist ein Verfahren zur Herstellung von Methionin oder einem Salz des Methionins, bei dem möglichst wenig Nebenprodukte entstehen sollen und dessen einzelne Komponenten sich gut abtrennen lassen sollen, insbesondere soll bei der Ausfällung eines Produktes dieses gut filtrierbar sein. Einzelne Komponenten sollen weitgehend recyclierbar bzw. regenerierbar sein.

Außerdem soll das erhaltene Methionin nur geringe Verfärbungen haben und gut lagerbeständig sein. Das Verfahren soll insbesondere im großtechnischen Maßstab sowie kontinuierlich möglich sein.

Erfindungsgemäß wird dies gelöst mit einem Verfahren zur Herstellung von Methionin oder einem Salz des Methionins durch Umsetzung der Komponenten 3-Methylmercaptopropionaldehyd, Cyanwasserstoff, Ammoniak und Kohlendioxid zum 5-(2-Methylmercaptoethyl)-hydantoin und weiterer Umsetzung desselben zum Methionin oder dessen Salz, wobei die Umsetzung der Komponenten über mindestens eine Vormischung derart eingeleitet wird, daß man ein erstes Gemisch bildet, bestehend aus 3-Methylmercaptopropionaldehyd und Cyanwasserstoff und daß man dieses erste Gemisch mit einer Lösung von Ammoniak und Kohlendioxid in Wasser zur reaktiven Umsetzung zum 5-(2-Methylmercaptoethyl)-hydantoin vereinigt.

Unter Komponenten, aus denen die vorgenannten Komponenten herstellbar sind, fallen z. B. Salze von Cyanwasserstoff, Ammoniak und Kohlendioxid, wie z. B. Natrium oder Kaliumcyanid, Ammoniumcarbonat oder -bicarbonat, Natrium oder Kaliumcarbonat oder -bicarbonat und natürlich insbesondere deren Lösungen in Wasser. Entsprechende Komponenten für 3-Methylmercaptopropionaldehyd sind Acrolein und Methylmercaptan. Grundsätzlich ist es bevorzugt, bei dieser Reaktion eventuell eingesetzte Metallsalze möglichst im Unterschuß einzusetzen, d. h., daß die Aldehydkomponente und/oder die Cyanidkomponente im stöchiometrischen Überschuß zu einer eventuell eingesetzten Metallionenkomponente ist. Besonders bevorzugt wird hier möglichst kein Metallsalz, wie z. B. Natrium oder Kaliumcyanid, eingesetzt sondern die oben genannten Verbindungen. Insbesondere bei einem kontinuierlichen Prozeß können aber zusätzlich andere Metallsalze, z. B. Katalysatoren o. ä., zugegen sein. Deren Dosierung fällt nicht unter die oben angegebene bevorzugte Metallionenbeschränkung hinsichtlich der Reaktionskomponenten.

Hier und im folgenden wird unter den angegebenen Komponenten auch deren unter den gegebenen Einsatzbedingungen veränderte Einsatzform verstanden. So liegt z. B. beim Einsatz von Ammoniak und Kohlendioxid in Wasser ein Teil dieser Komponenten als Ammonium(hydrogen)carbonat vor.

Nach diesem Verfahren wird das 5-(2-Methylmercaptoethyl)-hydantoin farblos mit praktisch quantitativer Ausbeute und so weitgehend frei von Verunreinigungen gewonnen, daß daraus ein Methionin bzw. dessen Salz in einem kontinuierlichen Prozeß unter Mutterlaugenrecyclierung erhalten werden kann, das sich durch außergewöhnliche Lagerstabilität bezüglich Verfärbung und Verklumpung auszeichnet.

Vorteilhaft ist es auch, wenn beim Beginn der Umsetzung alle Komponenten in ihrer vorgesehenen Einsatzmenge vollständig vorhanden sind, d. h., daß keine Komponente nachdosiert wird. Vorteilhaft ist es außerdem, wenn alle Komponenten in insgesamt zwei Vormischungen vereinigt werden, die dann zur Umsetzung miteinander vermischt werden.

Bei allen Umsetzungen ist es besonders günstig, wenn die einzelnen Komponenten bzw. Vormischungen schnell und möglichst innig miteinander vermischt werden.

Bevorzugt enthält das Reaktionsgemisch keine organischen Lösungsmittel, wenn organische Lösungsmittel eingesetzt werden, dann vorteilhaft zu weniger als 20 Gew.-Teile, insbesondere zu weniger als 10 Gew.-Teile, bezogen auf 100 Gew.-% Wasser.

Besonders vorteilhaft ist es, wenn die Vormischung(en) und ggf. einzelne Komponenten (es können eine oder mehrere Vormischungen miteinander zur Reaktion gebracht werden, wobei im Falle von zwei Vormischungen ggf. alle einzelnen Komponenten in diesen untergebracht sein können) in ein bereits erhaltenes Umsetzungsgemisch, enthaltend 5-(2-Methylmercaptoethyl)-hydantoin eingebracht werden. Hierbei ist es besonders vorteilhaft, wenn alle Vormischung(en) und ggf. einzelne Komponenten entweder benachbart oder mit einer zeitlichen (bei chargenweiser Herstellung) oder strömungsbedingten (bei kontinuierlicher Herstellung). Versetzung um höchstens 30 s in das

Umsetzungsgemisch eingeleitet werden.

Die Reaktion wird bei der oben beschriebenen Verfahrensweise vorzugsweise bei einer Temperatur über 80 °C ausgeführt; außerdem wird die Reaktion vorzugsweise bei Überdruck, besonders bevorzugt bei einem Druck größer als dem sich einstellenden Gleichgewichtsdruck (Reaktionsdruck), insbesondere bei mind. 3 bar und besonders vorteilhaft bei über 10 bar, durchgeführt.

Das oben beschriebene Verfahren mit seinen Varianten eignet sich insbesondere zur kontinuierlichen Durchführung.

Bei einem Verfahren zur Herstellung von Methionin oder einem Salz des Methionins sind insbesondere zwei der oben beschriebenen Verfahrensmaßnahmen für sich allein schon besonders vorteilhaft, so daß die Erfindung außerdem ein Verfahren zur kontinuierlichen Herstellung von Methionin oder einem Salz des Methionins betrifft, mit der Umsetzung der Komponenten 3-Methylmercaptopropionaldehyd, Cyanwasserstoff, Ammoniak und Kohlendioxid zum 5-(2-Methylmercaptoethyl)-hydantoin und weiterer Umsetzung desselben zum Methionin oder dessen Salz, wobei die Komponenten in ein Umsetzungsgemisch eingebracht werden, das aus den vorgenannten Komponenten gebildet ist und bereits mindestens 1/10 der theoretisch bildbaren Menge des 5-(2-Methylmercaptoethyl)-hydantoins enthält, und wobei die Umsetzung bei Überdruck, bevorzugt bei einem Druck von mindestens 3 bar, durchgeführt wird.

Hier ist es besonders vorteilhaft, wenn bei einem Druck von mind. 7 bar und insbesondere bei einem Druck von mind. 10 bar gearbeitet wird. Außerdem ist es besonders vorteilhaft, wenn die Komponenten unmittelbar oder mit einer strömungsbedingten Vorreaktionszeit von höchstens 30 s in das Umsetzungsgemisch eingeleitet werden, d. h., daß die einzelnen Komponenten höchstens 30 s in reaktivem Kontakt stehen, bevor sie in das Umsetzungsgemisch eingebracht werden.

Außerdem sind auch bei dieser Verfahrensvariante die oben beschriebenen einzelne vorteilhafte Maßnahmen durchführbar.

Die bis jetzt beschriebenen Verfahren werden besonders bevorzugt als ein Verfahren durchgeführt, wobei im nachfolgenden eine entsprechende summarische Darstellung dieses Verfahrens wiedergegeben wird. Die hierbei gegebenen weiteren Spezifikationen, z. B. hinsichtlich der Stöchiometrie, der Temperaturen, etc., gelten jeweils für sich allein auch vorteilhaft für die oben allgemein beschriebenen Verfahren.

Erfindungsgemäß wird das 5-(2-Methylmercaptoethyl)-hydantoin besonders vorteilhaft hergestellt, indem eine Lösung von Cyanwasserstoff in 3-Methylmercaptopropionaldehyd und eine Lösung von Ammoniak und Kohlendioxid in Wasser bereitet wird und diese Lösungen schnell und innig miteinander vermischt und zur Umsetzung gebracht werden. Ammoniak und Kohlendioxid können aus der Hydrolysestufe dieses Hydantoin zurückgeführt werden. Die Lösung von Cyanwasserstoff in 3-Methylmercaptopropionaldehyd wird zweckmäßigerweise so eingestellt, daß sie aus equimolaren Anteilen Cyanwasserstoff und 3-Methylmercaptopropionaldehyd besteht, oder aber überschüssige Anteile an Cyanwasserstoff enthält. Im allgemeinen ist es vorteilhaft, den Anteil Cyanwasserstoff in der Lösung nicht größer als 1.1 Mol je Mol 3-Methylmercaptopropionaldehyd zu wählen; vorzugsweise enthält die Lösung 1,005 bis 1,05 Mol Cyanwasserstoff je Mol 3-Methylmercaptopropionaldehyd.

Bei der Lösung von Ammoniak und Kohlendioxid in Wasser kann es sich um eine gesättigte oder verdünnte Lösung handeln; vorteilhaft ist der Gehalt an Ammoniak nicht unter etwa 5 Gew.-%. In der Lösung kann Ammoniumhydrogencarbonat, Ammoniumcarbonat, Carbaminsäure, Ammoniumcarbamidat, Cyansäure oder ein Gemisch aus diesen Komponenten vorliegen. Das Molverhältnis Ammoniak zu Kohlendioxid beträgt zweckmäßigerweise etwa 1,2 bis 4,0 Mol, vorzugsweise 1,6 bis 1,8 Mol Ammoniak je Mol Kohlendioxid. Die Lösung des Cyanwasserstoffs in 3-Methylmercaptopropionaldehyd wird mit der Lösung des Ammoniaks und Kohlendioxids in Wasser so vermischt, daß in der Mischung zweckmäßigerweise ein Molverhältnis Ammoniak zu 3-Methylmercaptopropionaldehyd von etwa 1,2 bis 6 zu 1,0, vorzugsweise 2,0 bis 4,0 zu 1,0, insbesondere 2,5 bis 3,0 zu 1,0, vorliegt.
Die Umsetzung wird bei Umgebungstemperatur oder darüber, zweckmäßigerweise bei Temperaturen über 60 °C, vorteilhaft etwa zwischen 80 °C und 140 °C, ausgeführt. Vorzugsweise werden Temperaturen zwischen 80 und 130 °C, insbesondere zwischen 90 und 120 °C, gewählt. Wenngleich die Umsetzung bei beliebigem Druck ablaufen kann, ist es zweckmäßig, bei erhöhtem Druck zu arbeiten; vorteilhaft erweisen sich Drücke bis 20 bar, insbesondere Drücke, die 2 bis 3 bar über dem Gleichgewichtsdruck des Umsetzungsgemisches liegen. Die Umsetzungszeit richtet sich nach den Umsetzungsbedingungen, insbesondere nach der Temperatur und nach den Mengenverhältnissen.

Bei der bevorzugten Arbeitsweise ist es besonders vorteilhaft, die Lösung des Cyanwasserstoffs in 3-Methylmercaptopropionaldehyd und die Lösung des Ammoniaks und Kohlendioxids im Wasser in ein Umsetzungsgemisch dieser Substanzen, d. h. in ein zuvor bei der Umsetzung der Lösungen entstandenes Gemisch, in dem ganz oder teilweise die Umsetzung vom Hydantoin erfolgt ist, einzutragen und in diesem Gemisch die Umsetzung auszuführen.

Besonders vorteilhaft ist es, eine kontinuierliche Arbeitsweise zu wählen, hierzu das Umsetzungsgemisch im Kreislauf zu führen, in diesem Kreislauf an zwei benachbarten Stellen stetig die Lösungen von Cyanwasserstoff in 3-Methylmercaptopropionaldehyd und vom Ammoniak und Kohlendioxid in Wasser einzuspeisen und an einer anderen Stelle stetig aus dem Kreislauf einen entsprechenden Anteil des Umsetzungsgemisches abzuziehen. Obwohl das Mischungsverhältnis zwischen den Lösungen, die in den Kreislauf eingespeist werden, und dem Umsetzungsgemisch, das im Kreislauf geführt wird, beliebig sein kann, ist es zweckmäßig, dieses Mischungsverhältnis so zu wählen, daß je Volumenteil der Lösungen mehrere Volumenteile des Umsetzungsgemisches, ggf. 1000 oder mehr, bevorzugt 5 bis 100, insbesondere 10 bis 25 Volumenteile des Umsetzungsgemisches, vorliegen. Von entscheidendem Vorteil ist es, die in den Kreislauf eingespeisten Lösungen mit dem im Kreislauf geführten Umsetzungsgemisch schnell und innig zu vermischen; dieses kann ggf. über eine Mischdüse, einen statischen Mischer, durch eine hohe Kreislaufzirkulation oder eine Kombination aller Maßnahmen bewerkstelligt werden. In den aus dem Kreislauf abgezogenen Anteilen des Umsetzungsgemisches ist die Umsetzung ggf. nicht vollständig abgelaufen, so daß es vorteilhaft sein kann, diese Anteile noch einige Zeit in einem Nachreaktor ausreagieren zu lassen. Falls weniger als 5 Volumenteile des Umsetzungsgemisches auf ein Volumenteil der Lösungen vorliegen, besteht die Gefahr der Polymerisation der Ausgangskomponenten. Das Gemisch färbt sich dunkel, es kommt zu Ausfällungen und damit zu Ausbeuteverlusten und/oder technischen Störungen.

Das wie oben beschrieben hergestellte, jedoch auch auf andere Art und Weise hergestelltes, 5-(2-Methylmercaptoethyl)-hydantoin kann weiter zu einem Alkalisalz des Methionins und ggf. weiter zum Methionin umgesetzt werden. Die Beschreibung umfaßt daher auch ein Verfahren zur Herstellung von Methionin oder eines Alkalisalzes von Methionin durch Hydrolyse von 5-(2-Methylmercaptoethyl)-hydantoin in Gegenwart einer wässrigen Lösung enthaltend Alkali und Kohlendioxid, und ggf. weitere Umsetzung zum Methionin, wobei die Hydrolyse zumindest zu Beginn in Gegenwart von mind. 0,1 eq, insbesondere bis 7 eq, Ammoniak je Equivalent 5-(2-Methylmercaptoethyl)-hydantoin durchgeführt wird.

Besonders vorteilhaft ist es, wenn beide Verfahren kombiniert werden.

Es hat sich gezeigt, daß es besonders vorteilhaft ist, wenn die Hydrolyse von Anfang an in Gegenwart von Alkali und Kohlendioxid, d. h., daß insbesondere eine Mischung von Alkaliverbindungen vorliegt, insbesondere Alkalihydrogencarbonat, Alkalicarbonat, Alkalihydroxid, wobei Alkali insbesondere für Kalium und Natrium steht, durchgeführt wird. Die Menge von Alkali und Kohlendioxid ist dabei zweckmäßig wenigstens die auf das Hydantoin bezogene stöchiometrische Menge. Diese kann nach oben deutlich überschritten werden. Ein Molverhältnis mit einem Überschuß von etwa 3 : 1 bezogen auf das Hydantoin ist besonders vorteilig; grundsätzlich ist davon auszugehen, daß ein noch größerer Überschuß noch günstiger ist. Für die Praxis jedoch sind Verhältnisse von etwa 1,5 : 1 - 2 : 1 besonders bevorzugt. Es wird dabei noch zusätzlich etwas Ammoniak zugegeben, der entsprechend ebenfalls teils auch in Form von Ammoniumverbindungen vorliegt. Besonders vorteilhaft ist es hierbei, wenn zu Beginn der Hydrolyse je Mol 5-(2-Methylmercaptoethyl)-hydantoin max. 7 Mol Ammoniak (incl. Ammoniumverbindungen) vorliegt. Hierdurch wird erreicht, daß die Hydrolyse praktisch ohne Nebenproduktbildung und in guten Ausbeuten abläuft und andererseits nicht oder nur wenig Alkalicarbonat ausfällt. Besonders vorteilhaft ist hierbei, wenn während der Hydrolyse Ammoniak und/oder Kohlendioxid, ggf. zusammen mit Wasser, aus dem Reaktionssystem ausgeschleust werden. Hierdurch lassen sich die Reaktionsbedingungen besonders günstig steuern, so daß weiterhin kein Alkalicarbonat ausfällt und die Reaktion vollständig verläuft.

Die Hydrolyseverfahren werden günstigerweise bei einer Temperatur von 120 bis 250 °C und entsprechend einem Druck von 5 bis 30 bar durchgeführt. In diesem Bereich ergeben sich sehr gute Umsetzungen und geringe Nebenproduktbildung. Vorteilhaft ist auch, wenn die Alkalikomponente zumindest equimolar bezüglich des 5-(2-Methylmercaptoethyl)-hydantoins eingesetzt wird. Man erhält dann neben der vollständigen Hydrolyse auch praktisch quantitativ das entsprechende Alkalisalz des Methionins. Bevorzugt enthält die Hydrolyselösung zu Beginn auch bereits Methionin bzw. dessen Salz, auch dies hat einen günstigen, vermutlich autokatalytischen Effekt auf die Hydrolyse.

Bei dieser Verfahrensweise kann vorteilhaft im Laufe bzw. nach der Hydrolyse praktisch das gesamte Ammoniak und das gesamte Kohlendioxid aus der Hydrolyselösung abgezogen werden, so daß das Hydrolysat im wesentlichen frei von Ammoniak und Kohlendioxid entnommen werden kann.

Auch hier ist es besonders vorteilhaft, das Verfahren kontinuierlich durchzuführen. Ganz besonders vorteilhaft ist hierbei, daß die bis jetzt beschriebenen Verfahren zusammengeschaltet werden können, insbesondere als gesamtkontinuierlicher Prozeß, wobei Kohlendioxid und Ammoniak recycliert werden können.

Im folgenden wird die Hydantoin-Hydrolyse näher beschrieben, wobei einzelne nähere Spezifikationen grundsätzlich für sich allein auch für die oben gegebene allgemeine Verfahrensweise gelten. Die im folgenden wiedergegebene Verfahrensweise wird für entsprechende Kaliumverbindungen beschrieben, da es sich hierbei um die am meisten bevorzugte Ausführungsform handelt.

Eine Kaliummethioninat-Lösung wird durch Hydrolyse von 5-(2-Methylmercaptoethyl)-hydantoin in Gegenwart von Kaliumhydroxid, Kaliumcarbonat und/oder Kaliumhydrogencarbonat oder eines Gemisches davon und bei Anwesenheit von überschüssigem Ammoniak, Kohlendioxid, Kohlensäure, Cyansäure oder eines Gemisches davon in Wasser bei einer Temperatur von 120 bis 250 °C und einem Druck von 5 bis 30 bar erhalten. Vorteilhaft erfolgt die Hydrolyse des Hydantoins bezogen auf das Hydantoin in Gegenwart von 1 bis 15 eq einer oder mehrerer Kaliumverbindungen (z. B. KOH, KHCO₃, K₂CO₃, Kaliummethioninat). Während bzw. nach der Hydrolyse ist es außerdem vorteilhaft, wenn entstehendes oder noch vorhandenes Ammoniak und/oder Kohlendioxid ganz oder teilweise aus dem Reaktionssystem abgetrennt wird. Grundsätzlich kann jedes 5-(2-Methylmercaptoethyl)-hydantoin eingesetzt werden, günstigerweise wird ein Hydantoin eingesetzt, das wie oben beschrieben erhältlich ist.

Vorteilhaft ist das Ammoniak zu Beginn der Hydrolyse ein Mol-Verhältnis zu Kohlendioxid von 1,1 bis 8,0. Günstig ist auch ein Mol-Verhältnis des Ammoniaks zum Hydantoin von 0,2 bis 5. Bei der beschriebenen Verfahrensweise ist es möglich, das Ammoniak und das Kohlendioxid unmittelbar aus dem oben beschriebenen Verfahren, der Hydantoin-Herstellung, zu übernehmen, so daß das Hydantoin unmittelbar aus der Hydantoin-Herstellung zusammen mit dem noch verbliebenen Ammoniak und Kohlendioxid in die Hydrolyse eingebracht werden kann, wobei hier dann noch eventuell gewünschte andere Ammoniak- und/oder Kohlendioxidkonzentrationen eingestellt werden können.

Bei dem hier beschriebenen Verfahren findet die Hydrolyse des 5-(2-Methylmercaptoethyl)-hydantoins bei Temperaturen von 120 - 250 °C, vorzugsweise von 150 - 200 °C, insbesondere von 160 - 180 °C, statt; der Druck während der Reaktion soll 5 - 30 bar, vorzugsweise 5 - 10 bar, insbesondere 7 - 9 bar, betragen.

Das Verfahren wird vorteilhaft in einer mit Dampf beheizten Kolonne mit Einbauten durchgeführt. Die 5-(2-Methylmercaptoethyl)-hydantoin-Lösung wird vorteilhaft am Kopf der Kolonne in der Geschwindigkeit kontinuierlich zugegeben, daß das Hydrolyseprodukt, Kaliummethioninatlösung, am Boden entsprechend abgezogen werden kann; d. h., die Hydrolyse am Boden der Kolonne quantitativ abgelaufen ist. Die gasförmigen Bestandteile, Wasserdampf, Ammoniak und Kohlendioxid, werden günstigerweise am Kopf der Kolonne ausgeschleust und können vorteilhaft zur Wiederherstellung der wässrigen Ammoniak/Kohlendioxid-Lösung zur Herstellung des 5-(2-Methylmercaptoethyl)-hydantoins eingesetzt werden.

Zur Hydrolyse des 5-(2-Methylmercaptoethyl)-hydantoins wird eine wäßrige Lösung aus Kaliumhydroxid, Kaliumcarbonat und/oder Kaliumhydrogencarbonat mit einem Kaliumionengehalt von vorteilhaft 100 - 200 g, vorzugsweise 140 - 160 g, Kalium pro Liter Hydrolyselösung eingesetzt. Zweckmäßigerweise wird an dieser Stelle bei einem kontinuierlichen Prozeß die Mutterlauge nach der Methionin-Feststoffabtrennung wieder eingesetzt; die Mutterlauge kann dann zusätzlich noch - der Löslichkeit entsprechend - Rest-Methionin enthalten, auch dies hat sich als günstig für das Verfahren herausgestellt.

Die mittlere Verweilzeit der Reaktionslösung in der Hydrolysekolonne beträgt vorteilhaft 10 - 20 Minuten. Das Molverhältnis der Kaliumionenmenge zu der Summe aus 5-(2-Methylmercaptoethyl)-hydantoin + Methionin beträgt günstig bis zu 10, vorzugsweise 1,3 - 5, insbesondere 1,5 - 2. Die erhaltene Ausbeute an Kaliummethioninat in der Reaktionslösung beträgt bei dieser Verfahrensweise typisch 99.0 - 100 %. Die Konzentration an Kaliummethioninat kann durch entsprechende Auswahl der Hydantoinkonzentration oder durch Verdünnen bzw. Einengen der nach der Hydrolyse anfallenden Lösung eingestellt werden.

Zu dem hier beschriebenen Verfahren gehört auch die Freisetzung von Methionin aus Alkalimethioninat, vorteilhaft aus einer wässrigen Lösung, wie sie nach den obigen Verfahrensweisen erhältlich ist, durch Freisetzung mit Kohlendioxid, wobei man der wässrigen Lösung, enthaltend das Alkalimethioninat, vor der Freisetzung des Methionins einen Entschäumer zugibt. Die Beschreibung enthält außerdem ein Verfahren zur Herstellung von Methionin aus Alkalimethioninat in wässriger Lösung durch Freisetzung mit Kohlendioxid, wobei man die Freisetzung in einem Rührzellenreaktor mit intensiver Durchmischung oder in einem Rührreaktor mit quasi idealer Durchmischung durchführt.

Diese beiden Verfahrensweisen werden vorzugsweise miteinander kombiniert. Als Entschäumer eignen sich alle Verbindungen, die eine schaumbremsende Funktion ausüben. Vorzugsweise wird der Entschäumer in Form einer Dispersion in die Lösung eingebracht. Hierdurch erhält man eine besonders gute Verteilung in der Lösung und nicht eine Konzentrierung im wesentlichen an der Oberfläche der wässrigen Lösung. Hierdurch wird der günstige Effekt des Entschäumers auf die Freisetzung des Methionins, insbesondere die Vermeidung der Bildung von dünnen Plättchen oder Schuppen, begünstigt. Es entstehen feste kugelige Kristalle, überwiegend mit einem Durchmesser von 100 bis 200 µm. Vorteilhaft wird der Entschäumer in einer Konzentration von 1 000 bis 10 000 ppm, bezogen auf das Gesamtmethionin (Methionin + Methioninat, umgerechnet auf Methionin) zugegeben.

Bei der Freisetzung des Methionins aus der wässrigen Lösung mittels Kohlendioxid ist es besonders vorteilhaft, wenn das Kohlendioxid über eine Düsenvorrichtung im Bereich des Bodens in die wäßrige Lösung eingeleitet wird. Dies begünstigt wiederum die Freisetzung des Methionins. Außerdem führt man die Freisetzung vorteilhaft bei einem Druck von 1 bis 30 bar durch, vorzugsweise auch bei einer Temperatur von 0 bis 100 °C.

Ganz besonders vorteilhaft wird eine wäßrige Lösung eingesetzt, die im wesentlichen frei von Ammoniak ist.

Auch die letzte Verfahrensweise wird besonders günstig kontinuierlich durchgeführt. Vorzugsweise wird die beschriebene Verfahrensweise mit der vorher beschriebenen Verfahrensweise zur Herstellung eines Alkalisalzes von Methionin kombiniert, wobei besonders bevorzugt die gesamte Kombination der oben beschriebenen Verfahrensweisen möglich ist.

Im folgenden wird das Verfahren zur Freisetzung von Methionin anhand des bevorzugten Kalium-D,L-Methioninats beschrieben, wobei auch andere Alkali, z. B. Natrium, möglich sind. Die hierbei angegebenen weiteren bevorzugten oder allgemeinen Verfahrensbedingungen gelten entsprechend auch bei der oben beschriebenen allgemeinen Verfahrensweise.

Bei der Freisetzung von D,L-Methionin aus Kalium-D,L-Methioninat durch Einleiten von Kohlendioxid insbesondere in eine Hydrolyse-Lösung des 5-(2-Methylmercaptoethyl)-hydantoins ist es besonders vorteilhaft, wenn die Lösung praktisch frei von Ammoniak ist. Vorzugsweise enthält die Lösung außerdem gelöstes D,L-Methionin. Außerdem können noch gewisse Mengen an Kaliumcarbonat und Kaliumhydrogencarbonat zugegen sein. Die Lösung kann, sofern gewünscht, vor der Kohlendioxid-Zugabe über Aktivkohle gereinigt werden. Die Kohlendioxid-Zugabe erfolgt üblicherweise bei einer Temperatur von 0 bis 100 °C, vorzugsweise bei 20 bis 35 °C, und üblicherweise bei einem Druck von 1 bis 30 bar, vorzugsweise von 2 bis 5 bar. Vorzugsweise wird das Kohlendioxid so lange in das Reaktionsgemisch eingeleitet, bis ein pH-Wert von ca. 7 bis 9, vorzugsweise 7,5 bis 8,5, erreicht wird und/oder bis die Ausfällung des D,L-Methionins beendet ist. Hierbei ist es besonders vorteilhaft, wenn das Kohlendioxid am Boden eines Reaktors direkt oder zweckmäßigerweise fein verteilt über eine Düsenvorrichtung eingeführt wird. Der Reaktor ist vorteilhaft als Rührzellenreaktor oder als quasi idealer Rührreaktor ausgebildet. Außerdem kann insbesondere bei einer kontinuierlichen Verfahrensweise der Entschäumer zusätzlich noch den Durchsatz erhöhen. Der Entschäumer wird üblicherweise in einer Menge von mindestens 1 000 und zweckmäßig bis 10 000 ppm, vorzugsweise 3 000 bis 5 000 ppm, bezogen auf das in der Reaktionslösung befindliche Gesamtmethionin, insbesondere als wäßrige Emulsion zudosiert. Das freigesetzte Methionin wird vorteilhaft von der Mutterlauge abgetrennt und ist nach dem Trocknen weitgehend staubarm und zeichnet sich durch gute Fließfähigkeit und ein hohes Schüttgewicht aus. Die Methioninpartikel besitzen überwiegend einen Durchmesser von 100 bis 200 µm. Bei dieser Verfahrensweise beträgt die Ausbeute des isolierten D,L-Methionins üblicherweise 98 bis 100 %. Die nach der D,L-Methionin-Abtrennung, insbesondere Filtration, erhaltene Mutterlauge kann vorteilhaft wieder zur Hydrolyse des 5-(2-Methylmercaptoethyl)-hydantoins, ggf. nach Konzentrierung und/oder Abzug von CO₂, eingesetzt werden.

Die Erfindung wird im folgenden anhand von Figuren und Beispielen näher ausgeführt.

Es zeigen
- Fig. 1: eine Verfahrensskizze zur kontinuierlichen Herstellung von 5-(2-Methylmercaptoethyl)-hydantoin;
- Fig. 2: eine Verfahrensskizze zur kontinuierlichen Hydrolyse von 5-(2-Methylmercaptoethyl)-hydantoin zu Alkalimethioninat, und
- Fig. 3: eine Verfahrensskizze zur kontinuierlichen Freisetzung und Isolierung von D,L-Methionin aus Alkali-D,L-Methioninat. (Fig. 2 und Fig. 3 gehören nicht einem Schutzbereich des Patents)

In den Figuren sind der Einfachheit halber Nebenkomponenten, wie Überschuß an NH₃/CO₂, z. B. bei der Ausschleusung, nicht aufgeführt.

### Beispiele 1 - 4

### Beispiele zur Herstellung von 5-(2-Methylmercaptoethyl)-hydantoin

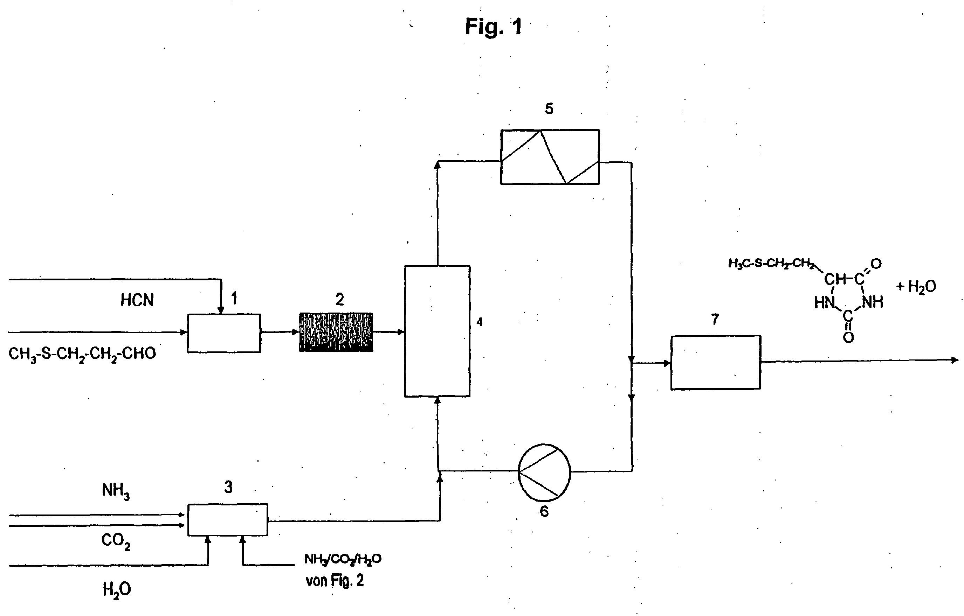

Der allgemeine Verfahrensablauf für die Beispiele 1 - 4 ist in Fig. 1 dargestellt. Bei der kontinuierlichen Arbeitsweise wird Cyanwasserstoff über eine Mischdüse 1 mit 3-Methylmercaptopropionaldehyd und einem nachgeschalteten Statikmixer 2 vermischt. In einem Mischreaktor 3 wird eine Lösung von Ammoniak und Kohlendioxid in Wasser hergestellt, wobei diese Komponenten von den nachfolgenden Stufen recycliert werden können. Die beiden Mischungen werden in einen Kreislaufreaktor in das Umsetzungsgemisch kontinuierlich eingespeist. In einer Mischeinrichtung 4 erfolgt eine gute momentane Durchmischung der Lösungen mit dem im Kreislauf geführten Umsetzungsgemisch. Die Umlaufmischung wird über einen Wärmetauscher 5, mit dem die gewünschte Temperatur eingestellt wird, gepumpt 6. Aus dem Kreislauf wird von dem Umsetzungsgemisch kontinuierlich ein entsprechender Anteil abgezogen. Dieser wird dann zur Vervollständigung der Reaktion einem Nachreaktor 7 zugeführt. Das so erhaltene Produktgemisch kann dann ggf. sofort in die nächste Reaktionsstufe überführt werden.

### Beispiel 1:

Es wurde eine Einrichtung wie in Fig. 1 verwendet. Zunächst wurde auf 90 °C angewärmtes Wasser im Kreislauf geführt; dann wurde in den Kreislauf stündlich eine Lösung von 10.5 Mol Cyanwasserstoff in 10 Mol 3-Methylmercaptopropionaldehyd und 6,8 l einer wässrigen Ammoniumcarbonatlösung, die 9.6 Gew.-% Ammoniak und 15.2 Gew.-% Kohlendioxid enthielt, eingespeist. Die Umwälzung im Kreislauf betrug 300 1 pro Stunde. Die Temperatur wurde auf 90 °C gehalten; der Druck betrug 14 bar. Aus dem Kreislauf wurde stetig ein dem Zufluß entsprechendes Volumen des Umsetzungsgemisches abgezogen und zur Nachreaktion geleitet. Die mittlere Verweilzeit war im Kreislauf 10 min und in der Nachreaktion 2 h. Die Ausbeute an 5-(2-Methylmercaptoethyl)-hydantoin, bzw. an auf Methionin verseifbaren Verbindungen, bezogen auf eingesetzten 3-Methylmercaptopropionaldehyd, betrug 99.8 %. (Die Angaben beziehen sich auf 3-Methylmercaptopropionaldehyd als 100 % gerechnet.)

### Beispiel 2:

Es wurde wie in Beispiel 1 verfahren, jedoch wurde zu Beginn statt Wasser eine bei Zimmertemperatur gesättigte Ammoniumcarbonatlösung im Kreislauf geführt, die 9.6 Gew.-% Ammoniak und 15.2 Gew.-% Kohlendioxid enthielt. Die Ausbeute betrug 99.7 %.

### Beispiel 3:

Es wurde wie nach Beispiel 1 verfahren, jedoch wurde die Temperatur im Kreislauf und bei der Nachreaktion auf 115 °C gehalten. Der Druck betrug 16 bar. Die Umwälzung im Kreislauf betrug 150 1 pro Stunde. Die mittlere Verweilzeit war im Kreislauf 6 min und in der Nachreaktion 20 min. Die erreichte Ausbeute betrug 99.9 %.

### Beispiel 4:

Es wurde wie in Beispiel 1 verfahren, jedoch wurden stündlich eine Lösung von 10.1 Mol Cyanwasserstoff in 10.0 mol 3-Methylmercaptopropionaldehyd und 6.8 l einer wässrigen Ammoniumcarbonatlösung, die 5.5 Gew.-% Ammoniak und 8.5 Gew.-% Kohlendioxid enthielt, eingespeist. Die Temperatur wurde im Kreislauf und bei der Nachreaktion auf 115 °C gehalten. Der Druck betrug 16 bar. Die Durchflußgeschwindigkeit im Kreislauf war 150 l pro Stunde; die mittlere Verweilzeit im Kreislauf war 6 min und in der Nachreaktion 40 min. Die Ausbeute betrug 99.8 %.

Die Beispiele 5-7 gehören nicht zum Schutzumfang des Patents.

### Beispiele zur Herstellung von Kalium-Methioninat-Lösung

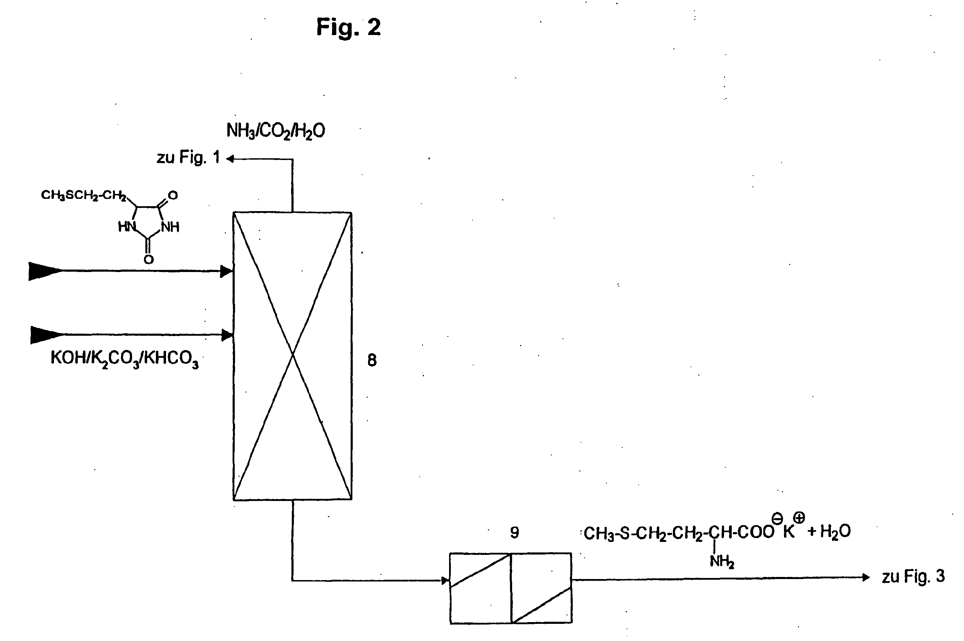

Der allgemeine Verfahrensablauf für die Herstellung von Kalium-Methioninat-Lösung ist in Fig. 2 wiedergegeben.

### Beispiel 5:

### Beispiel mit einer Druckapparatur ohne Zirkoneinbauten

Mittels Pumpendruck wird in eine kontinuierlich arbeitende Druckkolonne 8 aus Edelstahl (vgl. Fig. 2), die mit Dampf betrieben wird, stündlich eine Lösung von 100 kg Kaliumhydrogencarbonat in wäßrige Lösung und 41 kg 5-(2-Methylmercaptoethyl)-hydantoin in 400 l Wasser eingespeist. Das Reaktionsgemisch wird dabei auf 180 °C aufgeheizt und hat eine mittlere Verweilzeit von ca. 15 min bei etwa 8 bar. Freigesetztes Ammoniak und Kohlendioxid wird am Kopf der Reaktorkolonne über ein Druckhalteventil abgezogen. Am Boden des Druckreaktors wird die Reaktionslösung entspannt und mit dem Wärmetauscher 9 abgekühlt. Dabei werden stündlich 41.9 kg Kalium-Methioninat in Lösung gewonnen (94.5 % d. Th.).

### Beispiel 6:

Es wird eine Apparatur gemäß Fig. 2 verwendet, die eine Druckkolonne beinhaltet, welche Einbauten aus Zirkon besitzt.

Mit einer Pumpe werden stündlich 553 kg 5-(2-Methylmercaptoethyl)-hydantoin in 1600 1 Reaktionslösung aus der Hydantoinherstellung nach Abb. 1 und 3550 l eines Gemisches aus Kaliumcarbonat/ Kaliumhydrogencarbonat und Kaliumhydroxid in wässriger Lösung aus der Mutterlaugenrückführung nach der Methioninfeststoffabtrennung mit einem Kaliumgehalt von 140 g pro Liter und einem Rest-Methioningehalt von 120 g pro Liter auf den Kopf einer Druckhydrolysekolonne 8 mit Zirkoneinbauten eingespeist. Die Reaktionstemperatur beträgt 165 °C; der Reaktionsdruck beträgt 7 bar. Das freiwerdende Ammoniak und Kohlendioxid werden am Kopf der Kolonne über ein Druckhalteventil ausgeschleust und der 5-(2-Methylmercaptoethyl)-hydantoin-Synthese erneut zugeführt.
Am Sumpf der Druckapparatur werden 5150 l pro Stunde eines wässrigen Gemisches mit 96.5 g Kalium pro Liter und 175 g Methionin pro Liter (entsprechend einer Zunahme von 476 kg/h Methionin im Gesamtsystem) erhalten (Ausbeute: 100 %). Die Reaktionslösung wird über einen Wärmetauscher 9 abgekühlt und der Methionin-Freisetzung zugeführt.

### Beispiel 7

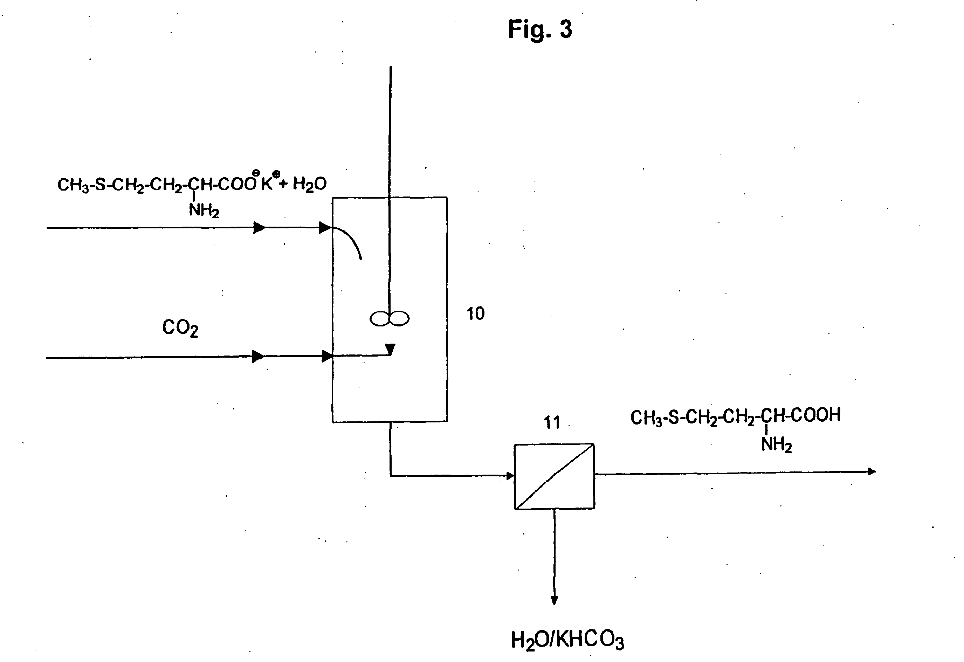

### Beispiel zur Freisetzung von D,L-Methionin aus Kalium-D,L-methioninat (Fig. 3)

Am Kopf eines Rührreaktors 10 von 340 1 Fassungsvermögen werden kontinuierlich 686 1 pro Stunde einer wässrigen Lösung mit 83,6 kg Kalium-D,L-methioninat (Hydrolyselösung von 5-(2-Methylmercaptoethyl)-hydantoin mit zusätzlich 39,77 kg Kreislauf-Methionin und Kaliumverbindungen eingespeist. Gleichzeitig wird am Reaktorboden Kohlendioxid zugeführt, so daß im Reaktor ein Druck von 2 - 3 bar herrscht. Ebenso werden 0,38 kg pro Stunde Entschäumer in Form einer wässrigen Emulsion in den Reaktor eindosiert; diese Menge entspricht ca. 3940 ppm Entschäumer pro Kilogramm Gesamt-Methionin. Die Reaktionstemperatur wird bei 25 °C gehalten. Um im Reaktor ein konstantes Niveau zu halten, werden am unteren Teil des Reaktors der Zudosierung entsprechende Mengen an Reaktionslösung ausgeschleust. Die Ausschleussuspension wird abfiltriert, wobei stündlich 66,5 kg Fest-D,L-Methionin (als Trockensubstanz gerechnet) erhalten werden und die Mutterlauge mit einem Restgehalt von 39,77 kg D,L-Methionin als Verseifungsagens in die Hydantoin-Hydrolysestufe rezykliert werden kann. Die Ausbeute ist quantitativ.

## Patentansprüche

1. Verfahren zur Herstellung von Methionin oder einem Salz des Methionins durch Umsetzung der Komponenten 3-Methylmercaptopropionaldehyd, Cyanwasserstoff, Ammoniak und Kohlendioxid zum 5-(2-Methylmercaptoethyl)-hydantoin und weiterer Umsetzung desselben zum Methionin oder dessen Salz, **dadurch gekennzeichnet, dass** die Umsetzung der Komponenten über mindestens eine Vormischung derart eingeleitet wird, dass man ein erstes Gemisch bildet, bestehend aus 3-Methylmercaptopropibnaldehyd und Cyanwasserstoff und dass man dieses erste Gemisch mit einer Lösung von Ammoniak und Kohlendioxyd in Wasser zur reaktiven Umsetzung zum 5-(2-Methylmercaptoethyl)-hydantoin vereinigt.

2. Verfahren nach Anspruch 1, **dadurch gekennzeichnet, dass** im ersten Gemisch der molare Anteil des Cyanwasserstoffs in der Lösung nicht größer als 1,1 Mol je Mol 3-Methylmercaptopropionaldehyd ist.

3. Verfahren nach einem der vorhergehenden Ansprüche, **dadurch gekennzeichnet, dass** alle Komponenten in insgesamt zwei Vormischungen eingesetzt werden

4. Verfahren nach Anspruch 3, **dadurch gekennzeichnet, dass** man die erste Vormischung mit einer Lösung von Ammoniak und Kohlendioxid in Wasser vermischt und zur Umsetzung bringt.

5. Verfahren gemäß Anspruch 1, **dadurch gekennzeichnet, dass** die Vormischungen in ein bereits erhaltenes Umsetzungsgemisch enthaltend 5-(2-Methylmercaptoethyl)-hydantoin eingebracht wird.

6. Verfahren gemäß Anspruch 1, **dadurch gekennzeichnet, dass** man die Lösung des Cyanwasserstoffs in 3-Methylmercaptopropionaldehyd mit der Lösung des Ammoniaks und Kohlendioxids in Wasser so vermischt, daß in der Mischung ein Molverhältnis Ammoniak zu 3-Methylmercaptopropionaldehyd von etwa 1,2 bis 6 zu 1,0 vorliegt.

7. Verfahren gemäß Anspruch 1, **dadurch gekennzeichnet, dass** man eine Lösung von Ammoniak und Kohlendioxid in Wasser einsetzt, wobei das Molverhältnis von Ammoniak zu Kohlendioxid sich auf 1,2 bis 4, 0 Mol Ammoniak je Mol Kohlendioxid beläuft.

8. verfahren nach einem der vorhergehenden Ansprüche, **dadurch gekennzeichnet, dass** die Umsetzung bei der Temperatur zwischen 80 und 140°C durchgeführt wird,

9. Verfahren nach einem der vorhergehenden Ansprüche, **dadurch gekennzeichnet, dass** es bei einem erhöhten Druck bis 20 bar durchgeführt wird.

10. Verfahren nach einem der vorhergehenden Ansprüche, **dadurch gekennzeichnet, dass** es kontinuierlich durchgeführt wird.

## Claims

1. Process for the preparation of methionine or a salt of methionine by conversion of the components 3-methylmercaptopropionaldehyde, hydrogen cyanide, ammonia and carbon dioxide to 5-(2-methyl-mercaptoethyl)hydantoin and further conversion thereof to methionine or its salt, **characterized in that** the conversion of the components is initiated via at least one premix so as to form a first mixture, consisting of 3-methylmercaptopropionaldehyde and hydrogen cyanide, and **in that** this first mixture is combined with a solution of ammonia and carbon dioxide in water for reactive conversion to the 5-(2-methylmercapto-ethyl)hydantoin.

2. Process according to Claim 1, **characterized in that**, in the first mixture, the molar fraction of the hydrogen cyanide in the solution is not greater than 1.1 mol per mole of 3-methylmercaptopropionaldehyde.

3. Process according to either of the preceding claims, **characterized in that** all the components are used in a total of two premixes.

4. Process according to Claim 3, **characterized in that** the first premix is mixed with a solution of ammonia and carbon dioxide in water and converted.

5. Process according to Claim 1, **characterized in that** the premixes are introduced into a previously obtained conversion mixture comprising 5-(2-methyl-mercaptoethyl) hydantoin.

6. Process according to Claim 1, **characterized in that** the solution of the hydrogen cyanide in 3-methylmercaptopropionaldehyde is mixed with the solution of ammonia and carbon dioxide in water such that the mixture comprises a molar ratio of ammonia:3-methylmercaptopropionaldehyde of about 1.2 to 6:1.0.

7. Process according to Claim 1, **characterized in that** a solution of ammonia and carbon dioxide in water is used wherein the molar ratio of ammonia:carbon dioxide is 1.2 to 4.0 mol of ammmonia per mole of carbon dioxide.

8. Process according to any one of the preceding claims, **characterized in that** the conversion is carried out at a temperature between 80 and 140°C.

9. Process according to any one of the preceding claims, **characterized in that** it is carried out at an elevated pressure of up to 20 bar.

10. Process according to any one of the preceding claims, **characterized in that** it is carried out as a continuous operation.

## Revendications

1. Procédé pour la préparation de méthionine ou d'un sel de méthionine par transformation des composants 3-méthylmercaptopropionaldéhyde, acide cyanhydrique, ammoniac et dioxyde de carbone en 5-(2-méthylmercaptoéthyl)-hydantoïne et transformation ultérieure de celle-ci en méthionine ou son sel, **caractérisé en ce que** la transformation des composants est démarrée via au moins un prémélange de telle manière qu'on forme un premier mélange constitué par du 3-méthylmercaptopropionaldéhyde et de l'acide cyanhydrique et on réunit ce premier mélange avec une solution d'ammoniac et de dioxyde de carbone dans l'eau pour la transformation par réaction en 5-(2-méthylmercaptoéthyl)-hydantoïne.

2. Procédé selon la revendication 1, **caractérisé en ce que** dans le premier mélange, la proportion molaire de l'acide cyanhydrique dans la solution n'est pas supérieure à 1,1 moles par mole de 3-méthylmercaptopropionaldéhyde.

3. Procédé selon l'une quelconque des revendications précédentes, **caractérisé en ce que** tous les composants sont utilisés dans au total deux prémélanges.

4. Procédé selon la revendication 3, **caractérisé en ce qu'**on mélange et qu'on amène à réagir le premier prémélange avec une solution d'ammoniac et de dioxyde de carbone dans l'eau.

5. Procédé selon la revendication 1, **caractérisé en ce que** les prémélanges sont introduits dans un mélange de transformation déjà produit contenant de la 5-(2-méthylmercaptoéthyl)-hydantoïne.

6. Procédé selon la revendication 1, **caractérisé en ce qu'**on mélange la solution de l'acide cyanhydrique dans le 3-méthylmercaptopropionaldéhyde avec la solution de l'ammoniac et du dioxyde de carbone dans l'eau de telle manière qu'on trouve dans le mélange un rapport molaire d'ammoniac à 3-méthylmercaptopropionaldéhyde d'environ 1,2 à 6:1, 0.

7. Procédé selon la revendication 1, **caractérisé en ce qu'**on utilise une solution d'ammoniac et de dioxyde de carbone dans l'eau, le rapport molaire d'ammoniac à dioxyde de carbone étant de 1,2 à 4,0 moles d'ammoniac par mole de dioxyde de carbone.

8. Procédé selon l'une quelconque des revendications précédentes, **caractérisé en ce que** la transformation est réalisée à une température entre 80 et 140°C.

9. Procédé selon l'une quelconque des revendications précédentes, **caractérisé en ce qu'**il est réalisé à une pression élevée jusqu'à 20 bars.

10. Procédé selon l'une quelconque des revendications précédentes, **caractérisé en ce qu'**il est réalisé en continu.
